# EUROPÄISCHE PATENTSCHRIFT

(11) **EP 3 598 010 B1**
(45) Veröffentlichungstag und Bekanntmachung des Hinweises auf die Patenterteilung: **09.10.2024**
(21) Anmeldenummer: 19180457.4
(22) Anmeldetag: 17.06.2019
(51) Int. Cl.: F24F 3/16, F24F 11/39, C12M 1/22, F24F 110/50, F24F 13/28

(54) **VORRICHTUNG ZUM ÖFFNEN UND/ODER SCHLIESSEN EINER PETRISCHALE ZUM MESSEN DER VERKEIMUNG EINES KONTROLLIERTEN RAUMES**
DEVICE FOR OPENING/CLOSING PETRIDISHES FOR MEASURING THE CONTAMINATION OF A CONTROLLED AREA
DISPOSITIF POUR OUVRIR/FERMER DES BOITES DE PETRI POUR LA MESURE DE PROLIFÉRATION DE GERMES D'UN ESPACE CONTRÔLÉ

(30) Priorität: 18.07.2018 DE 202018003348 U
(43) Veröffentlichungstag der Anmeldung: 22.01.2020
(73) Patentinhaber: ACCURRO GmbH, 35041 Marburg (DE)
(72) Erfinder: BATTENBERG, Ralf, 35037 Marburg (DE); WAGNER, Alexander, 35102 Lohra (DE)
(74) Vertreter: Walther Bayer Faber Patentanwälte PartGmbB

(56) Entgegenhaltungen:
- DE-T2- 69 624 821
- JP-A- H11 225 743
- US-A1- 2004 151 617
- US-A1- 2015 075 301

## Beschreibung

Die vorliegende Erfindung betrifft eine Vorrichtung zum Öffnen und/oder Schließen einer Petrischale, wobei die Petrischale zum Messen der Verkeimung eines kontrollierten Raumes, insbesondere eines Reinraumes, gemäß dem Oberbegriff des Anspruches 1 verwendet wird.

In vielen Fällen soll die Herstellung von Medikamenten in einer sterilen und/oder keimfreien Umgebung erfolgen, weshalb derartige Medikamente in kontrollierten Räumen hergestellt, verarbeitet und/oder verpackt werden. In diesen kontrollierten Räumen wird die Keimbelastung durch geeignete Maßnahmen entsprechend den Anforderungen reduziert. Bei kontrollierten Räumen mit einer extrem niedrigen Keimbelastung spricht man auch von Reinräumen.

Während der Herstellung, der Verarbeitung und/oder der Verpackung der Medikamente muss aus Gründen der Qualitätssicherung nachgewiesen werden, dass die zulässige Keimbelastung in dem kontrollierten Raum nicht überschritten wurde. Hierzu wird in dem kontrollierten Raum für eine bestimmte Zeit, zum Beispiel für 20 Minuten, eine Keime identifizierende Nährlösung ausgesetzt. Dies geschieht dadurch, dass vor Beginn der Verarbeitung des Mediamentes in dem kontrollierten Raum eine Anzahl von eine geeignete Nährlösung tragenden Petrischalen platziert wird. Es versteht sich, dass die Petrischalen zu diesem Zeitpunkt noch keimdicht verschlossen sind. Nachdem der kontrollierte Raum geschlossen wurde und nachdem die Keimbelastung im kontrollierten Raum auf das gewünschte Maß reduziert und nachdem mit der Verarbeitung des Medikamentes begonnen wurde, wird eine erste Petrischale geöffnet. Dies geschieht dadurch, dass ein Bediener vorzugsweise über einen in einer Wand des kontrollierten Raumes vorhandenen, keimdichten Handschuh, den Deckel der ersten Petrischale ergreift, abhebt und beiseite legt, so dass die im Boden der Petrischale befindliche Nährlösung nun eventuell im abgeschlossenen Raum befindliche Keime aufnehmen kann. Nach einer vorgegebenen Zeit, zum Beispiel 20 Minuten, wird der Bediener, wieder unter Nutzung des keimfreien Handschuhs, den Deckel der Petrischale aufnehmen und auf den die Nährlösung tragenden Boden der Petrischale aufsetzen und damit die Nährlösung keimdicht abschließen. Zu gegebener Zeit wird auf die gleiche Art und Weise eine zweite Petrischale geöffnet und nach der vorgegebenen Zeit wieder geschlossen. Dabei werden entsprechend den durch die Qualitätsstandards vorgegebenen Bedingungen entsprechend viele Petrischalen auf die oben beschriebene Weise geöffnet und wieder geschlossen.

Nachdem die Herstellung, Verarbeitung und/oder Verpackung der Medikamente abgeschlossen ist, werden die wieder keimdicht verschlossenen Petrischalen aus dem nunmehr offenen, kontrollierten Raum herausgeholt und in ein Labor verbracht. Dort werden die sich in der Nährlösung befindlichen Keime ausgewertet.

Es versteht sich, dass es für den Bediener sehr schwierig ist, unter Verwendung des keimfeien Handschuhs den vergleichsweise kleinen Deckel der Petrischale zu ergreifen, um die Petrischale zu öffnen und ordnungsgemäß wieder zu schließen. Für den Fall, dass der Deckel nicht richtig auf dem Boden aufsitzt, besteht die Gefahr, dass die in diesem Zyklus hergestellten Medikamente unverkäuflich sind, weil die erforderliche Keimfreiheit nicht nachgewiesen werden kann. Außerdem ist das Öffnen und Schließen der Petrischale durch einen Bediener sehr kostenintensiv. Eine Vorrichtung zum Beschichten von Petrischalen ist in der DE 696 24 821 T2 offenbart.

Davon ausgehend liegt der vorliegenden Erfindung die Aufgabe zugrunde, eine Vorrichtung zum Öffnen und/oder Schließen einer Petrischale, wobei die Petrischale zum Messen der Verkeimung eines kontrollierten Raumes, insbesondere eines Reinraumes, verwendet wird, der eingangs genannten Art zu schaffen, mit der das Öffnen und/oder Schließen der Petrischale sehr viel zuverlässiger und kostengünstiger zu erreichen ist.

Als technische Lösung dieser Aufgabe wird erfindungsgemäß eine Vorrichtung zum Messen der Verkeimung eines kontrollierten Raumes mit den Merkmalen des Anspruches 1 vorgeschlagen. Vorteilhafte Weiterbildungen dieser Vorrichtung sind den jeweiligen Unteransprüchen zu entnehmen.

Eine nach dieser technischen Lehre ausgebildete Vorrichtung zum Öffnen und/oder Schließen einer Petrischale, wobei die Petrischale zum Vermessen der Verkeimung eines kontrollierten Raumes dient, hat den Vorteil, dass der Deckelhalter angehoben und/oder verschwenkt werden kann, ohne dass der Bediener den Deckel der Petrischale mühsam ergreifen muss. Vielmehr wird die Petrischale mit ihrem Deckel vor Beginn der Herstellung, Verarbeitung oder Verpackung der Medikamente, das heißt, vor Beginn der Entkeimung des kontrollierten Raumes in die erfindungsgemäße Vorrichtung zum Messen der Verkeimung eines kontrollierten Raumes gestellt und während der Herstellung und/oder der Verpackung des Medikamentes kann dann der Deckel zu gegebener Zeit von einem Bediener außerhalb des kontrollierten Raumes geöffnet und auch wieder geschlossen werden. Hierdurch entfällt das mühsame Ergreifen des Deckels mit dem keimfreien Handschuh, was einerseits eine wesentliche Arbeitserleichterung und damit Kostenersparnis darstellt und andererseits ein zuverlässiges Öffnen und vor allem Schließen der Petrischale zur Folge hat, sodass deutlich weniger Ausfälle beim Messen der Verkeimung auftreten.

Erfindungsgemäß ist an der Petrischalenplattform eine Bodenaufnahme vorgesehen, die den Boden der Petrischale in einer definierten Position aufnimmt, sodass auch der Deckel der Petrischale eine definierte Position einimmt. Dies hat den Vorteil, dass der Bediender die Petrischale stets in der richtigen Position platziert, ohne hierzu viel Aufmerksamkeit aufbringen zu müssen.

Erfindungsgemäß wird die gewählte Position des Bodens in der Bodenaufnahme dadurch erreicht, dass an der Bodenaufnahme mindestens eine Anlageschulter ausgebildet ist, an der der Boden der Petrischale anlegbar ist.

In einer anderen, bevorzugten Ausführungsform weist die Bodenaufnahme mindestens zwei sich gegenüberliegende Anlageschultern auf, sodass der Boden zwischen den beiden sich gegenüberliegenden Anlageschultern form- und/oder kraftschlüssig gehalten werden kann. Dabei hat es sich als vorteilhaft erwiesen, die Anlageschultern korrespondierend zur Petrischale kreissegmentförmig gebogen auszubilden, sodass die Petrischale in diesen gebogenen Anlageschultern definiert und verrutschsicher gehalten ist. Des Weiteren hat es sich als vorteilhaft erwiesen, die Anlageschultern aus einem formstabilen, aber nachgiebigen Material zu bilden. Dies hat den Vorteil, dass beim Einschieben der Petrischale in die Vorrichtung zum Messen der Verkeimung eines kontrollierten Raumes eine oder mehrere Anlageschultern zumindest teilweise verdrängt werden, und dass die verdrängten Anlageschultern wieder in ihre Ausgangsposition zurückfinden, sobald der Boden der Petrischale in der definierten Position angekommen ist. Hierdurch ist es mit geringem Kraftaufwand möglich, die Petrischale an den Anlageschultern vorbei in die Bodenaufnahme zu bringen und den Boden formschlüssig in der Bodenaufnahme zu halten.

In einer weiteren, ebenfalls bevorzugten Ausführungsform sind die sich gegenüberliegenden Anlageschultern korrespondierend zur Petrischale so angeordnet, dass der Boden der Petrischale beim Einführen der Petrischale in die Plattform diese Anlageschultern teilweise verdrängt und sodass die Anlageschulter auch dann verdrängt bleibt, wenn der Boden seine definierte Position erreicht hat. Durch das Streben der Anlageschulter in die Ausgangsposition zurückzugelangen, erfolgt eine kraftschlüssige Halterung des Bodens in der Bodenaufnahme. Es versteht sich, dass eine solche kraftschlüssige Aufnahme sowohl bei kreissegmentförmig gebogenen Anlageschultern, als auch bei nicht kreissegmentförmig gebogenen Anlageschultern möglich ist.

In einer anderen, ebenfalls bevorzugten Ausführungsform weist die Deckelaufnahme zwei sich gegenüberliegende Halteelemente auf, zwischen denen der Deckel der Petrischale formschlüssig und/oder kraftschlüssig gehalten werden kann. Dies hat den Vorteil, dass der in der Deckelaufnahme gehaltene Deckel der Petrischale durch Verschwenken oder Verdrehen die Deckelaufnahme vom Boden der Petrischale wegbewegt werden kann, sodass sich die Petrischale öffnet. Analoges gilt beim Schließen der Petrischale. Ein großer Vorteil besteht nun darin, dass nicht mehr der Deckel der Petrischale, sondern lediglich die Deckelaufnahme bewegt werden muss, um die Petrischale zu öffnen oder zu schließen. Im Ergebnis ist dies sehr viel einfacher zu bewerkstelligen und führt zu einem zuverlässigeren Schließen der Petrischale.

Dabei hat es sich als vorteilhaft erwiesen, die Halteelemente aus einem formstabilen, aber nachgiebigem Material auszubilden und derart korrespondierend zum Deckel der Petrischale anzuordnen, dass der Deckel der Petrischale die Halteelemente beim Eintritt in die Deckelaufnahme zumindest teilweise verdrängt, um den Deckel formschlüssig und/oder kraftschlüssig in der Deckelaufnahme zu halten. Dies hat den Vorteil, dass der Deckel lediglich durch Bewegen der Deckelaufnahme geöffnet und geschlossen werden kann, was zu einer einfachen Handhabung und zu einem zuverlässigen Schließen der Petrischale führt.

In einer vorteilhaften Weiterbildung sind die Halteelemente derart aus einem formstabilen aber nachgiebigen Material gebildet und derart korrespondierend zu der Petrischale an der Deckelhalterung angeordnet, dass der Deckel der Petrischale die Halteelemente beim Eintritt in die Deckelaufnahme zumindest teilweise verdrängt. Nachdem der Deckel in seiner endgültigen Position angekommen ist, gelangen die Halteelemente wieder in ihre ursprüngliche Position, um den Deckel formschlüssig in der Deckelaufnahme zu halten. In einer anderen, vorteilhaften Ausführungsform sind die Halteelemente aus einem formstabilen, aber nachgiebigem Material gebildet und derart korrespondierend zum Deckel der Petrischale an der Deckelhalterung angeordnet, dass der Deckel der Petrischale die Halteelemente beim Eintritt in die Deckelaufnahme zumindest teilweise verdrängt, um den Deckel nach Erreichen der engültigen Position kraftschlüssig in der Deckelaufnahme zu halten. Dabei werden die Halteelemente während des Eintritts des Deckels in die Deckelaufnahme teilweise verdrängt und üben durch ihre dem Material innewohnende Rückstellkraft eine Haltekraft auf den Deckel aus, sodass der Deckel kraftschlüssig gehalten wird.

In einer besonders bevorzugten Ausführungsform sind die Halteelemente korrespondierend zum Deckel der Petrischale kreissegmentförmig gebogen ausgebildet und insbesondere aus einem formstabilen, aber nachgiebigem Material gebildet, dass der Deckel der Petrischale die Halteelemente beim Eintritt des Deckels in die Deckelhalterung zumindest teilweise verdrängt, wobei die Halteelemente diese in ihre Ausgangslage zurückdrängen und dabei den Deckel in seiner endgültigen Position sowohl formschlüssig als auch kraftschlüssig halten.

In einer anderen, bevorzugten Ausführungsform sind die Anlageschultern und/oder die Halteelemente derart angeordnet, dass die Petrischale bei geschlossenem Deckelhalter horizontal in die Petrischalenplattform hineingeschoben werden kann oder horizontal aus der Petrischalenplattform herausgenommen werden kann. Dies hat den Vorteil, dass die Petrischale in geschlossenem Zustand in der Petrischalenplattform plaziert werden kann, sodass die in der Petrischale befindliche Nährlösung zur Messung der Verkeimung des kontrollierten Raumes zur Verfügung steht. Ein weiterer Vorteil besteht darin, dass die Petrischalenplattform zwecks Hineinschieben oder Herausnehmen der Petrischale nicht betätigt werden muss, was die Handhabung stark vereinfacht. Noch ein weiterer Vorteil besteht darin, dass beim Hineinschieben der Petrischale in die Petrischalenplattform gleichzeitig auch der Boden und der Deckel der Petrischale in die entsprechenden Anlageschultern dieser Halteelemente gedrückt wird, dass die Petrischale anschließend zu gegebener Zeit geöffnet und wieder geschlossen werden kann.

In einer anderen, ganz besonders bevorzugten Ausführungsform umfassen die Mittel zum Anheben und/oder Verschwenken des Deckelhalters gegenüber dem Boden der Petrischale einen Elektroantrieb, insbesondere einen Stellmotor, eine von außerhalb des Raumes bedienbare Steuerungseinheit zur Steuerung des Elektroantriebes und eine sowohl am Elektroantrieb, als auch am Deckelhalter angreifende Betätigungsvorrichtung zum Anlegen und/oder Verschwenken des Deckelhalters. Dies hat den Vorteil, dass die Steuerung und damit auch der Elektroantrieb z. B. über W-LAN, Bluetooth oder auf andere Weise von außerhalb des kontrollierten Raumes bedient werden kann. Daraus ergibt sich der große Vorteil, dass einerseits eine manuelle Bedienung der Petrischalenplattform entfällt, was eine große Kostenersparnis zur Folge hat und wodurch ein zuverlässiges Schließen der Petrischale erreicht wird.

In einer anderen, ebenfalls besonders bevorzugten Ausführungsform umfassen die Mittel zum Anheben und/oder Verschwenken des Deckels in den Deckelhalter gegenüber der Petrischale einen Elektroantrieb, insbesondere einen Stellmotor, eine programmierbare Steuereinheit zur Steuerung des Elektromotors und eine sowohl am Elektroantrieb als auch am Deckelhalter angreifende Betätigungsvorrichtung zum Anheben und/oder Verschwenken des Deckelhalters. Diese Ausführungsform hat dieselben Vorteile wie in der zuletzt genannten Ausführungsform, kann aber zusätzlich noch derart programmiert werden, dass die Petrischale zu einer vorbestimmten Zeit geöffnet und geschlossen wird, sodass jegliche Bedienung entfällt, was zu einer weiteren Kostenersparnis führt.

In einer vorteilhaften Weiterbildung weist die Betätigungsvorrichtung zum Anheben und/oder Verschwenken des Deckelhalters einen verschwenkbar am Elektroantrieb angebrachten Betätigungshebel auf, an dem drehbeweglich eine Betätigungsstange gehalten ist, wobei die Betätigungsstange drehbeweglich an einem an der Deckelhalterung angebrachten Bürzel gehalten ist, wobei die Deckelhalterung schwenkbar an einer Schwenkachse gehalten ist und wobei der Bürzel über die Schwenkachse hinausstehend an der Deckelhalterung angebracht ist.

Diese Betätigungsvorrichtung ist einfach in der Herstellung, wartungsarm und zuverlässig im Betrieb.

Weitere Vorteile der erfindungsgemäßen Vorrichtung zum Messen der Verkeimung eines kontrolierten Raumes ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine perspektivische Darstellung eines kontrollierten Raumes mit einer ersten Ausführungsform einer erfindungsgemäßen Vorrichtung, wobei ein Bediener diese Vorrichtung von außerhalb des kontrollierten Raumes mittels einer drahtlosen Fernsteuerung bedient;
- Fig. 2: eine Seitenansicht der erfindungsgemäßen Vorrichtung gemäß Fig. 1 mit darin aufgenommener Petrischale und mit geschlossenem Deckelhalter;
- Fig. 3: eine Frontansicht der erfindungsgemäßen Vorrichtung gemäß Fig. 2 mit darin aufgenommener Petrischale und mit geöffnetem Deckelhalter;
- Fig. 4: eine Seitenansicht der erfindungsgemäßen Vorrichtung gemäß Fig. 1 mit darin aufgenommener Petrischale und mit geöffnetem Deckelhalter;
- Fig. 5: eine geschnitten dargestellte Draufsicht der erfindungsgemäßen Vorrichtung gemäß Fig. 3 mit darin aufgenommener Petrischale und mit geöffnetem Deckelhalter, geschnitten entlang Linie V - V in Fig. 3;

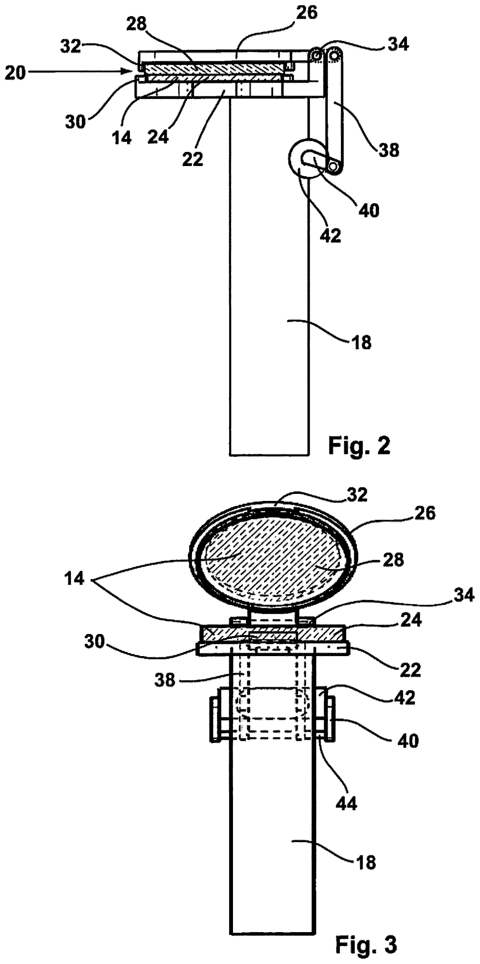

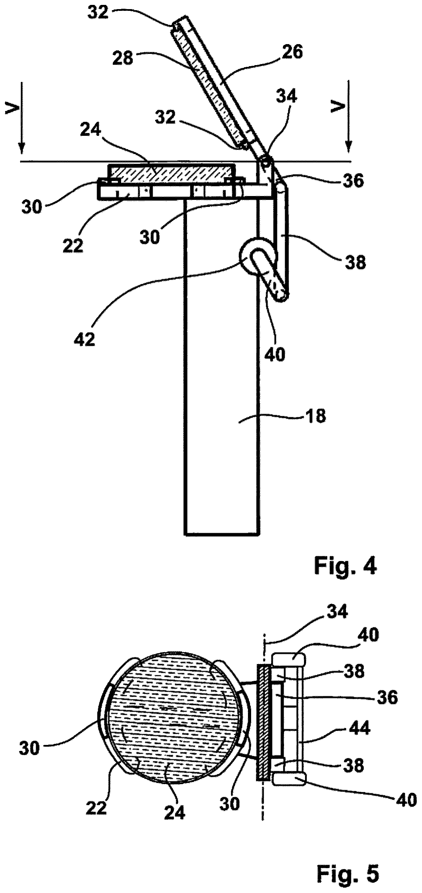

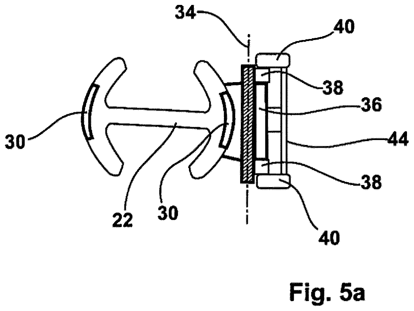

- Fig. 5 a: eine geschnitten dargestellte Draufsicht der erfindungsgemäßen Vorrichtung gemäß Fig. 3 ohne Petrischale und mit geöffnetem Deckelhalter, geschnitten entlang Linie V - V in Fig. 3.

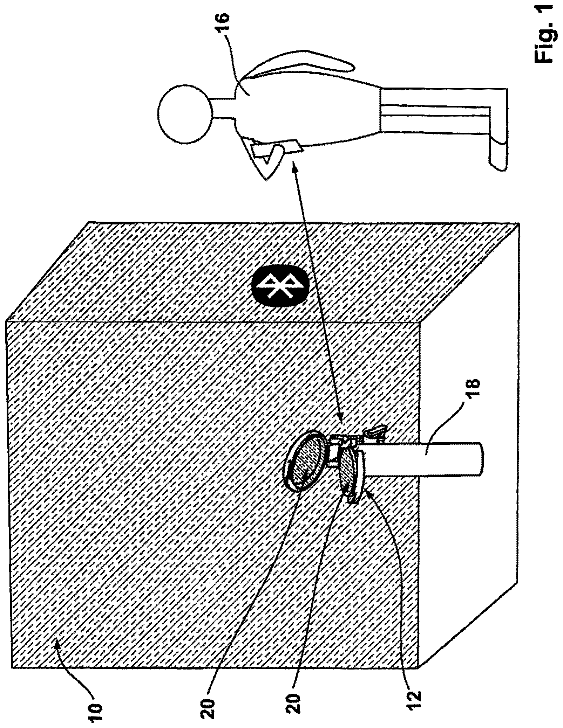

In Fig. 1 ist stilisiert ein kontrollierter Raum 10, hier ein Reinraum, dargestellt, der nach außen hin keimdicht verschlossen ist. In diesem kontrolliertem Raum 10 werden in einer sterilen oder keimarmen Umgebung, beispielsweise Medikamente hergestellt, weiterverarbeitet und/oder verpackt. Zur Messung der aktuell vorhandenen Anzahl von Keimen in diesem kontrollierten Raum 10 wird an einer geeigneten Stelle die erfindungsgemäße Vorrichtung 12 zum Messen der Verkeimung eines kontrollierten Raumes 10 aufgestellt und mit einer Petrischale bestückt, in der sich eine zur Aufnahme der Keime geeignete Nährlösung befindet.

Diese Vorrichtung zum Messen der Verkeimung kann z. B. über eine Bluetooth-Verbindung von einem Bediener 16 außerhalb des Raumes 10 geöffnet oder geschlossen werden.

In den Figuren 2 bis 5 und 5a ist diese Vorrichtung 12 zum Messen der Verkeimung detaillierter dargestellt. Diese Vorrichtung 12 zum Messen der Verkeimung umfasst einen Halteturm 18, auf dem eine Petrischalenplattform 20 angebracht ist. Diese Petrischalenplattform 20 umfasst eine Bodenaufnahme 22 zur Aufnahme des Bodens 24 der Petrischale 14 und einen schwenkbar am Halteturm 18 gehaltenen Deckelhalter 26 zum Halten eines Deckels 28 der Petrischale 14.

An der Bodenaufnahme 22 sind zwei sich gegenüberliegende Anlageschultern 30 nach oben abstehend ausgebildet, die kreissegmentförmig gebogen sind und derart korrespondierend zum Boden 24 der Petrischale 14 angeordnet sind, dass dieser Boden 24 formschlüssig und/oder kraftschlüssig in einer definierten Position an der Bodenaufnahme 22 gehalten ist. Dabei ist die Anlageschulter 30 aus einem formstabilen aber nachgiebigem Material gebildet, sodass die Petrischale 14 beim horizontalen Hineinschieben in die Petrischalenplattform 20 diese Anlageschultern 30 um ein gewisses Maß verdrängt, sodass der Boden 24 der Petrischale 14 bis in die dafür vorgesehene Position in der Bodenaufnahme 22 gelangt. Nachdem der Boden 24 in dieser Position angekommen ist, bewegen sich die Anlageschultern 30 zumindest teilweise aufgrund der dem Material innewohnenden Rückstellkräfte zumindest teilweise in die Anfangsstellung zurück und drücken dabei auf den Boden 24 der Petrischale 14, um diesen kraftschlüssig und/oder formschlüssig in Position zu halten.

Am Deckelhalter 26 sind zwei sich gegenüberliegende Halteelemente 32 ausgebildet, die, analog zu den Anlageschultern 30, ebenfalls aus einem formstabilen aber nachgiebigem Material hergestellt sind und die sich beim Hineinschieben des Deckels 28 der Petrischale 14 in die Petrischalenplattform 20 ebenfalls soweit verdrängen lassen, dass der Deckel 28 der Petrischale 14 in die dafür vorgesehen Position am Deckelhalter 26 gelangt. Anschließend drücken die dem Halteelement 32 innewohnenden Rückstellkräfte auf den Deckel 28 und halten diesen somit kraftschlüssig in der gewünschten Position.

Die Anlageschultern 30 und Halteelemente 32 sind so dimensioniert, dass die Petrischale 14 bei geschlossenem Deckelhalter 26 an den Anlageschultern 30 und den Halteelementen 32 vorbeigedrückt werden kann und dabei die Anlageschultern 30 und die Halteelemente 32 ein gewisses Stück verdrängt, sodass die Petrischale 14 bis in die dafür vorgesehene Position innerhalb der Petrischalenplattform 20 gelangen kann. Es versteht sich, dass die Petrischale 14 dabei in horizontaler Richtung in die Petrischalenplattform 20 geschoben wird. Analoges gilt beim Herausnehmen der Petrischale 14 aus der Petrischalenplattform 20, nur in umgekehrter Richtung.

Der Deckelhalter 26 ist über eine Schwenkachse 34 verschwenkbar an der Petrischalenplattform 20 gehalten. An der über die Schwenkachse 34 hinausstehenden Teil des Deckelhalters 26 ist ein Bürzel 36 ausgebildet, an dem eine rechte und eine linke Betätigungsstange 38 schwenkbar gehalten ist. Am anderen Ende der Betätigungsstange 38 ist ein Betätigungshebel 40 schwenkbar gehalten, der mit einem als Schrittmotor ausgeführten Elektroantrieb 42 wirkverbunden ist. Außerdem sind die beiden Betätigungshebel 40 über eine Verbindungsstange 44 miteinander verbunden.

Der Elektroantrieb 42 wird von einer hier nicht dargestellten Steuerungseinheit gesteuert und derart betätigt, dass zu gegebener Zeit der Deckelhalter 26 verschwenkt wird, um den Deckel 28 der Petrischale 14 vom Boden 24 der Petrischale 14 abzuheben und diese zu öffnen und der zu gegebener Zeit den Deckelhalter 26 zurückschwenkt, um den Deckel 28 der Petrischale 14 auf den Boden 24 der Petrischale 14 zu plazieren, um die Petrischale 14 keimdicht zu verschließen.

Die Steuerungseinheit ist dabei sowohl als programmierbare Steuereinheit ausgebildet, sodass entsprechend der Programmierung der Deckelhalter zu einer bestimmten Zeit hochgeschwenkt und zu einer bestimmten Zeit wieder zurückgeschwenkt wird, als auch über eine Fernbedienung von außerhalb des kontrollierten Raumes 10, z. B. über eine Bluetooth-Verbindung steuerbar ist. In anderen, hier nicht dargestellten Ausführungsformen, kann die Steuereinheit auch auf einem anderen Wege von außen bedient werden, z. B. über eine Kabelverbindung, über eine W-LAN Verbindung oder dergleichen.

Der Ablauf des Verfahrens zum Messen der Verkeimung eines kontrollierten Raumes, insbesondere eines Reinraumes, läuft wie folgt ab:
Zunächst wird mindestens eine, vorzugsweise mehrere Vorrichtungen 12 zum Messen der Verkeimung in dem kontrollierten Raum aufgestellt, wobei der Deckelhalter 26 eine geschlossene Position einnimmt. Anschließend wird jede Vorrichtung 12 zum Messen der Verkeimung mit einer keimdicht geschlossenen Petrischale 14 bestückt, indem die Petrischale 14 in horizontaler Richtung seitlich in die Petrischalenplattform 20 eingeschoben wird. Dabei werden die Anlageschultern 30 der Bodenaufnahme 22 und die Halteelemente 32 des Deckelhalters 26 soweit verdrängt, dass die Petrischale 14 an den Anlageschultern 30 und den Halteelementen 32 vorbeikommt und in die gewünschte, definierte Position in der Bodenaufnahme 22 gelangt, bis der Boden 24 der Petrischale 14 passgenau an den Anlageschultern 30 anliegt. Zumindest die Halteelemente 32 wurden beim Einschieben des Deckels 28 in die Petrischalenplattform 20 ein wenig verdrängt und drücken nun aufgrund der ihrem Material innewohnenden Rückstellkräfte auf den Deckel 28 und halten diesen kraftschlüssig. Gleichzeitig ist der Deckel 28 aber auch entsprechend der kreissegmentförmig gebogenen Halteelemente 32 formschlüssig am Deckelhalter 26 gehalten. Gleiches gilt auch für die Anlageschultern 30, die ebenfalls kreissegmentförmig gebogen sind und den Boden 24 formschlüssig halten.

Nachdem sämtliche Vorrichtungen 12 zum Messen der Verkeimung mit jeweils einer eine Nährlösung aufweisenden Petrischale 14 bestückt sind, verlässt der Bediener den kontrollierten Raum und der kontrollierte Raum wird dann auf die übliche Weise sterilisiert und entkeimt. Sobald die Verkeimung auf das für die Segmente erforderliche Maß reduziert worden ist, kann mit der Herstellung, der Verarbeitung und/oder der Verpackung der Medikamente begonnen werden. Während der Verarbeitung der Medikamente wird dann eine erste Vorrichtung 12 zum Messen der Verkeimung, z. B. durch eine Bluetooth-Fernbedienung betätigt, sodass der Deckelhalter 26 den Deckel 28 verschwenkt und dabei vom Boden 24 ablöst, sodass die in der Petrischale 14 befindliche Nährlösung nun Kontakt zum kontrollierten Raum erhält und sich im kontrollierten Raum befindliche Keime in der Nährlösung ansammeln können. Nach einer vorgegebenen Zeit kann der Bediener dann wiederum über die Bluetooth-Verbindung die Steuereinheit aktivieren, um den Deckelhalter 26 in die Ursprungsstellung zurückzubewegen, sodass der Deckel 28 auf den Boden 24 gesetzt wird und damit die Petrischale 14 keimfrei verschließt. Dieser Vorgang kann mit den anderen im kontrollierten Raum befindlichen Vorrichtungen 12 zum Messen der Verkeimung entsprechend wiederholt werden. Zu gegebener Zeit wird dann die Bearbeitung der Medikamente beendet und der Bediener holt die keimfrei verschlossenen Petrischalen 14 aus den Vorrichtungen 12 zum Messen der Verkeimung heraus, indem der die Petrischale 14 in horizontaler Richtung aus der Petrischalenplattform 20 herausdrückt und danach werden die Petrischalen14 in ein Labor zur Ermittlung der in der jeweiligen Petrischale 14 befindlichen Verkeimung gebracht. Sollte sich im Nachhinein herausstellen, dass eine Petrischale 14 mehr Keime als zulässig aufweist, wird das während dieser Zeit verarbeitete Medikament als unbrauchbar gekennzeichnet.

Alternativ kann das Öffen und Schließen der Petrischale auch in der Steuerung programmiert werden, sodass der gesamte Prozess auch ohne zutun des Bedieners abläuft.

## Patentansprüche

1. Vorrichtung zum Öffnen und/oder Schließen einer Petrischale,
wobei die Petrischale zum Messen der Verkeimung eines kontrollierten Raumes (10), insbesondere eines Reinraumes verwendet wird, umfassend eine Petrischalenplattform (20) zur Aufnahme einer einen Boden (24) und einen Deckel (28) umfassenden Petrischale (14),
Wobei die Petrischalenplattform (20) einen Deckelhalter (26) zum zuverlässigen Anheben und/oder Verschwenken des Deckels (28) der Petrischale (14) aufweist und dass an der Petrischalenplattform (20) Mittel zum Anheben und/oder Verschwenken des den Deckel (28) haltenden Deckelhaltes (26) gegenüber des Bodens der Petrischale (14) vorgesehen sind, die von außerhalb des kontrollierten Raumes (10) bedienbar sind,
**dadurch gekennzeichnet,**
**dass** die Petrischalenplattform (20) eine Bodenaufnahme (22) zur definierten Aufnahme des Bodens (24) der Petrischale (14) aufweist und die Bodenaufnahme (22) mindestens eine Anlageschulter (30) aufweist, an die der Boden (24) der Petrischale (14) anlegbar ist.

2. Vorrichtung nach Anspruch 1,
**dadurch gekennzeichnet,**
**dass** die Bodenaufnahme (22) mindestens zwei sich gegenüberliegende Anlageschultern (30) aufweist, zwischen denen der Boden (24) der Petrischale (14) formschlüssig und/oder kraftschlüssig gehalten werden kann.

3. Vorrichtung nach einem der Ansprüche 1 oder 2,
**dadurch gekennzeichnet,**
**dass** die Anlageschultern (30) korrespondierend zur Petrischale (14) kreissegmentförmig gebogen ausgebildet sind.

4. Vorrichtung nach einem der vorherigen Ansprüche,
**dadurch gekennzeichnet,**
**dass** die Anlageschultern (30) aus einem formstabilen, aber nachgiebigen Material gebildet sind, sodass der Boden (24) der Petrischale (14) die Anlageschultern (30) beim Eintritt des Bodens (24) in die Bodenaufnahme (22) zumindest teilweise verdrängt, um in der definierten Position formschlüssig und/oder kraftschlüssig in der Bodenaufnahme (22) gehalten zu werden.

5. Vorrichtung nach wenigstens einem der vorangehenden Ansprüche,
**dadurch gekennzeichnet,**
**dass** die Deckelaufnahme mindestens zwei sich gegenüberliegende Halteelemente (32) aufweist, die derart korrespondierend zum Deckel (28) der Petrischale (14) an der Deckelaufnahme gehalten sind, dass der Deckel (28) der Petrischale (14) von den Halteelementen (32) formschlüssig und/oder kraftschlüssig gehalten werden kann.

6. Vorrichtung nach Anspruch 5,
**dadurch gekennzeichnet,**
**dass** die Halteelemente (32) aus einem formstabilen, aber nachgiebigen Material gebildet sind und derart korrespondierend zum Deckel (28) der Petrischale (14) an der Deckelhalterung (26) angeordnet sind, dass der Deckel (28) der Petrischale (14) die Halteelemente (32) beim Eintritt in die Deckelaufnahme zumindest teilweise verdrängt, um den Deckel (28) formschlüssig und/oder kraftschlüssig in der Deckelaufnahme zu halten.

7. Vorrichtung nach Anspruch 5 oder 6,
**dadurch gekennzeichnet,**
**dass** die Halteelemente (32) korrespondierend zum Deckel (28) der Petrischale (14) kreissegmentförmig gebogen ausgebildet sind.

8. Vorrichtung nach wenigstens einem der vorangehenden Ansprüche,
**dadurch gekennzeichnet,**
**dass** die Anlageschultern (30) und/oder die Halteelemente (32) derart angeordnet sind, dass die Petrischale bei geschlossenem Deckelhalter (26) horizontal in die Petrischalenplattform (20) hineingeschoben werden kann oder horizontal aus der Petrischalenplattform (20) herausgenommen werden kann.

9. Vorrichtung nach wenigstens einem der vorangehenden Ansprüche,
**dadurch gekennzeichnet,**
**dass** die Mittel zum Anheben und/oder Verschwenken des den Deckel (28) haltenden Deckelhalters (26) gegenüber dem Boden (24) der Petrischale (14) einen Elektroantrieb (42) umfassen.

10. Vorrichtung nach wenigstens einem der vorangehenden Ansprüche,
**dadurch gekennzeichnet,**
**dass** die mittel zum Anheben und/oder Verschwenken des den Deckel (28) haltendenden Deckelhalters (26) gegenüber dem Boden (24) der Petrischale (14) einen Stellmotor, eine von außerhalb des Raumes bedienbare Steuerungseinheit und/oder eine programmierbare Steuerungseinheit zur Steuerung des Elektroantriebes (42) umfassen und eine sowohl am Elektroantrieb (42), als auch am Deckelhalter (26) angreifende Betätigungsvorrichtung zum Anheben und/oder Verschwenken des Deckelhalters (26).

11. Vorrichtung nach Anspruch 10,
**dadurch gekennzeichnet,**
**dass** die Betätigungsvorrichtung zum Anheben und/oder Verschwenken des Deckelhalters (26) einen verschwenkbar am Elektroantrieb (42) angebrachten Betätigungshebel (40) aufweist, an dem drehbeweglich eine Betätigungsstange (38) gehalten ist, dass die Betätigungsstange (38) drehbeweglich an einem an der Deckelhalterung (26) angebrachten Bürzel (36) gehalten ist, dass die Deckelhalterung (26) schwenkbar an einer Schwenkachse (34) gehalten ist und dass der Bürzel (36) über die Schwenkachse (34) hinausstehend an der Deckelhalterung (26) angebracht ist.

## Claims

1. An apparatus for opening and/or closing a Petri dish, wherein the Petri dish is used to measure the contamination of a controlled space (10), in particular of a clean room, comprising a Petri dish platform (20) for the reception of a Petri dish (14) comprising a base (24) and a cover (28),
wherein the Petri dish platform (20) has a cover holder (26) for the reliable raising and/or pivoting of the cover (28) of the Petri dish (14); and wherein means for raising and/or pivoting the cover holder (26) holding the cover (28) with respect to the base of the Petri dish (14) are provided that can be operated from outside the controlled space (10),
**characterized in that**
the Petri dish platform (20) has a base receiver (22) for the defined reception of the base (24) of the Petri dish (14) and the base receiver (22) has at least one abutment shoulder (30) at which the base (24) of the Petri dish (14) can be placed.

2. An apparatus in accordance with claim 1,
**characterized in that**
the base receiver (22) has at least two oppositely disposed abutment shoulders (30) between which the base (24) of the Petri dish (14) can be held with a shape match or a force fit.

3. An apparatus in accordance with one of the claims 1 or 2,
**characterized in that**
the abutment shoulders (30) are configured as curved in circular segment form corresponding to the Petri dish (14).

4. An apparatus in accordance with one of the preceding claims,
**characterized in that**
the abutment shoulders (30) are formed from a shape-stable, but yielding material so that the base (24) of the Petri dish (14) at least partly displaces the abutment shoulders (30) on entry of the base (24) into the base receiver (22) to be held in the base receiver (22) in the defined position with a shape match or a force fit.

5. An apparatus in accordance with at least one of the preceding claims,
**characterized in that**
the cover receiver has at least two oppositely disposed holding elements (32) that are held at the cover receiver corresponding to the cover (28) of the Petri dish (14) so that the cover (28) of the Petri dish (14) can be held with a shape match or a force fit by the holding elements (32).

6. An apparatus in accordance with claim 5,
**characterized in that**
the holding elements (32) are formed from a shape-stable, but yielding material and are arranged at the cover holder (26) corresponding to the cover (28) of the Petri dish (14) such that the cover (28) of the Petri dish (14) at least partly displaces the holding elements (32) on entry into the cover receiver to hold the cover (28) in the cover receiver with a shape match or a force fit.

7. An apparatus in accordance with claim 5 or claim 6,
**characterized in that**
the holding elements (32) are configured as curved in circular segment form corresponding to the cover (28) of the Petri dish (14).

8. An apparatus in accordance with at least one of the preceding claims,
**characterized in that**
the abutment shoulders (30) and/or the holding elements (32) are arranged such that the Petri dish (14) can be horizontally pushed into the Petri dish platform (20) with a closed cover holder (26) or can be horizontally removed from the Petri dish platform (20).

9. An apparatus in accordance with at least one of the preceding claims,
**characterized in that**
the means for raising and/or pivoting the cover holder (26) holding the cover (28) with respect to the base (24) of the Petri dish (14) comprise an electric drive (42).

10. An apparatus in accordance with at least one of the preceding claims,
**characterized in that**
the means for raising and/or pivoting the cover holder (26) holding the cover (28) with respect to the base (24) of the Petri dish (14) comprise a servomotor, a control unit operable from outside the space, and/or a programmable control unit for the control of the electric drive (42) and an actuation device engaging both at the electric drive (42) and at the cover holder (26) for raising and/or pivoting the cover holder (26).

11. An apparatus in accordance with claim 10,
**characterized in that**
the actuation device for raising and/or pivoting the cover holder (26) has an actuation lever (40) that is pivotably attached to the electric drive (42) and at which an actuation rod (38) is rotationally movably held; **in that** the actuation rod (38) is rotationally movably held at a nose (36) attached to the cover holder (26); **in that** the cover holder (26) is pivotably held at a pivot axle (34); and **in that** the nose (36) is attached to the cover holder (34) in a manner projecting over the pivot axle (34).

## Revendications

1. Dispositif pour ouvrir et/ou fermer une boîte de Petri, la boîte de Petri étant utilisée pour mesurer la prolifération de germes d'un espace contrôlé (10), en particulier une salle blanche, comprenant une plateforme pour boîte de Petri (20) destinée à recevoir une boîte de Petri (14) comprenant un fond (24) et un couvercle (28),
la plateforme pour boîte de Petri (20) présentant un support de couvercle (26) destiné au soulèvement et/ou au pivotement fiable du couvercle (28) de la boîte de Petri (14) et des moyens pour soulever et/ou pivoter le support de couvercle (26) maintenant le couvercle (28) par rapport au fond de la boîte de Petri (14) sont prévus sur la plateforme pour boîte de Petri (20), lesdits moyens pouvant être commandés depuis l'extérieur de l'espace contrôlé (10),
**caractérisé en ce que**
la plateforme pour boîte de Petri (20) présente un logement pour fond (22) destiné à recevoir de manière définie le fond (24) de la boîte de Petri (14) et le logement pour fond (22) présente au moins un épaulement d'appui (30), contre lequel le fond (24) de la boîte de Petri (14) peut être appliqué.

2. Dispositif selon la revendication 1,
**caractérisé en ce que**
le logement pour fond (22) présente au moins deux épaulements d'appui (30) opposés l'un à l'autre, entre lesquels le fond (24) de la boîte de Petri (14) peut être maintenu par complémentarité de forme et/ou par adhérence.

3. Dispositif de mesure selon une des revendications 1 ou 2,
**caractérisé en ce que**
les épaulements d'appui (30) sont réalisés courbés en forme de segment de cercle de façon à correspondre à la boîte de Petri (14).

4. Dispositif selon l'une des revendications précédentes,
**caractérisé en ce que**
les épaulements d'appui (30) sont formés en un matériau indéformable mais résilient, de telle sorte que le fond (24) de la boîte de Petri (14) déplace au moins en partie les épaulements d'appui (30) lors de l'entrée du fond (24) dans le logement pour fond (22) afin d'être maintenu dans la position définie dans le logement pour fond (22) par complémentarité de forme et/ou par adhérence.

5. Dispositif selon au moins une des revendications précédentes,
**caractérisé en ce que**
le logement pour couvercle présente au moins deux éléments de maintien (32) opposés l'un à l'autre, qui sont maintenus sur le logement pour couvercle de façon à correspondre au couvercle (28) de la boîte de Petri (14) de telle manière que le couvercle (28) de la boîte de Petri (14) peut être maintenu par complémentarité de forme et/ou par adhérence par les éléments de maintien (32).

6. Dispositif selon la revendication 5,
**caractérisé en ce que**
les éléments de maintien (32) sont formés en un matériau indéformable mais résilient et sont agencés sur le support de couvercle (26) de façon à correspondre au couvercle (28) de la boîte de Petri (14) de telle manière que le couvercle (28) de la boîte de Petri (14) déplace au moins en partie les éléments de maintien (32) lors de l'entrée dans le logement pour couvercle afin de maintenir le couvercle (28) dans le logement pour couvercle par complémentarité de forme et/ou par adhérence.

7. Dispositif selon la revendication 5 ou 6,
**caractérisé en ce que**
les éléments de maintien (32) sont réalisés courbés en forme de segment de cercle de façon à correspondre au couvercle (28) de la boîte de Petri (14).

8. Dispositif selon au moins une des revendications précédentes,
**caractérisé en ce que**
les épaulements d'appui (30) et/ou les éléments de maintien (32) sont agencés de telle manière que, lorsque le support de couvercle (26) est fermé, la boîte de Petri peut être introduite horizontalement dans la plateforme pour boîte de Petri (20) ou peut être retirée horizontalement de la plateforme pour boîte de Petri (20).

9. Dispositif selon au moins une des revendications précédentes,
**caractérisé en ce que**
les moyens pour soulever et/ou pivoter le support de couvercle (26) maintenant le couvercle (28) par rapport au fond (24) de la boîte de Petri (14) comprennent un entraînement électrique (42).

10. Dispositif selon au moins une des revendications précédentes,
**caractérisé en ce que**
les moyens pour soulever et/ou pivoter le support de couvercle (26) maintenant le couvercle (28) par rapport au fond (24) de la boîte de Petri (14) comprennent un moteur de commande, une unité de commande utilisable depuis l'extérieur de l'espace et/ou une unité de commande programmable destinée à la commande de l'entraînement électrique (42) ainsi qu'un dispositif d'actionnement agissant aussi bien sur l'entraînement électrique (42) que sur le support de couvercle (26) pour soulever et/ou pivoter le support de couvercle (26).

11. Dispositif selon la revendication 10,
**caractérisé en ce que**
le dispositif d'actionnement pour soulever et/ou pivoter le support de couvercle (26) présente un levier d'actionnement (40) monté pivotant sur l'entraînement électrique (42), sur lequel une barre d'actionnement (38) est maintenue mobile en rotation, **en ce que** la barre d'actionnement (38) est maintenue mobile en rotation sur un appendice (36) monté sur le support de couvercle (26), **en ce que** le support de couvercle (26) est maintenu pivotant sur un axe de pivotement (34) et **en ce que** l'appendice (36) est monté sur le support de couvercle (26) de manière à faire saillie au-delà de l'axe de pivotement (34).
